(19) Europäisches Patentamt
European Patent Office
Office européen des brevets

(11) **EP 2 346 420 B1**

(12) **EUROPEAN PATENT SPECIFICATION**

(45) Date of publication and mention
of the grant of the patent:
**13.04.2016 Bulletin 2016/15**

(51) Int Cl.:
*A61B 17/00* (2006.01)    *A61B 17/34* (2006.01)
*A61M 29/00* (2006.01)    *A61M 25/00* (2006.01)
*A61B 1/00* (2006.01)    *A61M 25/01* (2006.01)
*A61B 90/00* (2016.01)    *A61B 34/00* (2016.01)

(21) Application number: **09744458.2**

(22) Date of filing: **12.10.2009**

(86) International application number:
**PCT/IB2009/054474**

(87) International publication number:
**WO 2010/044051 (22.04.2010 Gazette 2010/16)**

(54) **INTERLOCKING NESTED CANNULA**

INEINANDERGREIFENDE VERSCHACHTELTE KANÜLE

CANULE EMBOÎTÉE D INTERVERROUILLAGE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **17.10.2008 US 106287**

(43) Date of publication of application:
**27.07.2011 Bulletin 2011/30**

(73) Proprietor: **Koninklijke Philips N.V.
5656 AE Eindhoven (NL)**

(72) Inventors:
• **GREENBLATT, Elliot Eliyahu
Briarcliff Manor
New York 10510-8001 (US)**
• **TROVATO, Karen Irene
Briarcliff Manor
New York 10510-8001 (US)**

• **POPOVIC, Aleksandra
Briarcliff Manor
New York 10510-8001 (US)**
• **STANTON, Douglas
Briarcliff Manor
New York 10510-8001 (US)**

(74) Representative: **van Velzen, Maaike Mathilde
Philips Intellectual Property & Standards
High Tech Campus 5
5656 AE Eindhoven (NL)**

(56) References cited:
WO-A1-2008/032230    WO-A2-2007/008332
WO-A2-2007/059233    US-A- 4 150 673
US-A1- 2003 199 916    US-A1- 2006 047 222
US-A1- 2007 010 843

**Description**

The present invention generally relates to nested cannula design and configurations that are customized for a patient to facilitate minimally invasive surgical procedures. The present invention specifically relates to a cannula interlocking mechanism that facilitates a fixed relative orientation of the telescoping tubes to each other.

International application WO 2007/008332 A2 discloses apparatuses and methods for performing minimally invasive medical procedures. One disclosed apparatus includes an outer elongate body including a lumen extending from a proximal portion to a distal portion of the outer elongate body. An inner elongate body is slidably received within the lumen of the outer elongate body. A distal portion of the inner elongate body is configures to be inserted into a tissue. The outer elongate body and the inner elongate body collectively define a channel to removably receive a tool when the outer elongate body and the inner elongate body are slidably extended from each other. At least one of the tool and the inner elongate body are configured to puncture the tissue.

International application WO 2007/059233 A2 discloses a surgical needle that is capable of following a complex path through cavities and tissue within a patient's anatomy. The needle has a plurality of overlapping flexible tubes, each of which has a pre-formed curvature and a pre-determined flexibility. Each of the plurality of flexible tubes is selected based on their respective pre-formed curvature and flexibility so that a given overlap configuration causes the combination of overlapping flexible tubes to form a predetermined shape that substantially matches a desired path through the anatomy. By individually controlling the translation and angular orientation of each of the flexible tubes, the surgical needle may be guided through the anatomy according to the desired path.

United States patent application US 2003/0199916 A1 discloses a multi-sheath delivery catheter and method for introducing a prosthesis into a boy lumen. It further discloses a delivery catheter having a mechanism for sequentially retracting concentric tubes to deploy the prosthesis. In another aspect, it discloses a mechanism for preventing relative axial movement of concentric tubes. In yet another aspect, it discloses a delivery system for introducing a prosthesis to a body lumen including a nose cone having a hole formed through it that is adapted to communicate with both the interior of the catheter and the ambient environment before insertion of the nose cone into the body lumen.

International Application WO 2008/032230 entitled "Active Cannula Configuration for Minimally Invasive Surgery" to Karen I. Trovato teaches systems and methods related to nested cannula design and configurations that are customized for a patient to facilitate minimally invasive surgical procedures. Generally, the nested cannulas design is created for a specific patient based on a pre-acquired 3D image of a particular anatomical region of the patient, and an identification of a target location within the anatomical region.

Specifically, nested cannulas (or a nested cannula configuration) are designed by utilizing the 3D image to generate a series of arc and straight shapes from a particular position and orientation in the 3D image of the anatomical region. The generated arc and straight shapes are utilized to calculate a pathway between an entry location and the target location. The generated pathway is utilized to generate a plurality of nested telescoping tubes that are configured and dimensioned with pre-set curved shapes. The tubes are typically extended largest to smallest, and the planner specification defines the lengths and the relative orientations between successive tubes to reach the target location.

The tubes are fabricated from a material exhibiting desirable levels of flexibility/elasticity. For example, the material may be Nitinol, which has superelastic properties that allow the Nitinol to bend when a force is applied and to return to its original shape once the force is removed. The tubes should maintain a relative orientation to each other when fully extended to comply with the generated pathway.

Tubes with circular cross sections have proven to be potentially unstable for certain configurations of the tubes. For example, long thin tubes with circular cross section may exhibit instability when curvatures of two (2) adjacent tubes are oriented at 180 degrees. In this case, movement of the tubes (e.g., for example due to vibration or extension through other curved shapes) may cause a sudden 'snap', where the tubes suddenly lose their 180 degree relative orientation. This uncontrolled movement may significantly deviate the tubes from the desired pathway and can damage tissue. Additionally, even in orientations other than 180 degrees, the tubes may twist relative to one another and cause inconsistent orientation.

The present invention is premised on an interlocking of telescoping tubes to facilitate a consistent relative orientation throughout the nested tubes that is preserved as the tubes are being extended. This ensures that the orientation set by the pathway planner can be achieved by the tubes.

One form of the present invention is an interlocking nested cannula set having a plurality of interlocking telescoping tubes cooperatively configured and dimensioned to reach a target location relative to an anatomical region. In this set, each tube has an interlocking shape. Additionally, a nesting of an inner tube within an outer tube includes a gap between the tubes for allowing a rotation of the inner tube within the outer tube that is limited by the outer tube and a rotation of the outer tube about the inner tube that is limited by the inner tube, wherein the inner tube and the outer tube interlock within the gap to limit the rotation of the inner tube and the outer tube relative to the gap.

Another form of the present invention is a nested cannula system employing a pathway planner for designing a plurality of interlocking telescoping tubes configured and dimensioned to reach a target location relative to an anatomical region. In this system, each tube has an interlocking shape. Additionally, a nesting of an inner tube within an outer tube includes a gap between the tubes for allowing a rotation of the inner tube within the outer tube that is limited by the outer tube and a rotation of the outer tube about the inner tube that is limited by the inner tube, wherein the inner tube and the outer tube interlock within the gap to limit the rotation of the inner tube and the outer tube relative to the gap.

The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims and equivalents thereof.

FIG. 1. illustrates an exemplary pair of interlocking tubes in accordance with the present invention prior to the inner tube being nested within the outer tube.
FIGS. 2-4 illustrates the interlocking principle of the present invention.
FIG. 5 illustrates a first exemplary interlocking of the tubes shown in FIG. 1 in accordance with the present invention.
FIG. 6 illustrates a second exemplary pair of interlocking of the tubes shown in FIG. 1 in accordance with the present invention.
FIGS. 7-20 illustrate various interlocking shapes in accordance with the present invention.
FIG. 21 illustrates an exemplary embodiment of a nested cannula system in accordance with the present invention.
FIG. 22 illustrates an exemplary 3-D neighborhood of arcs representing a nested cannula set of interlocking telescoping tubes in accordance with the present invention having pre-set shapes and curvatures.

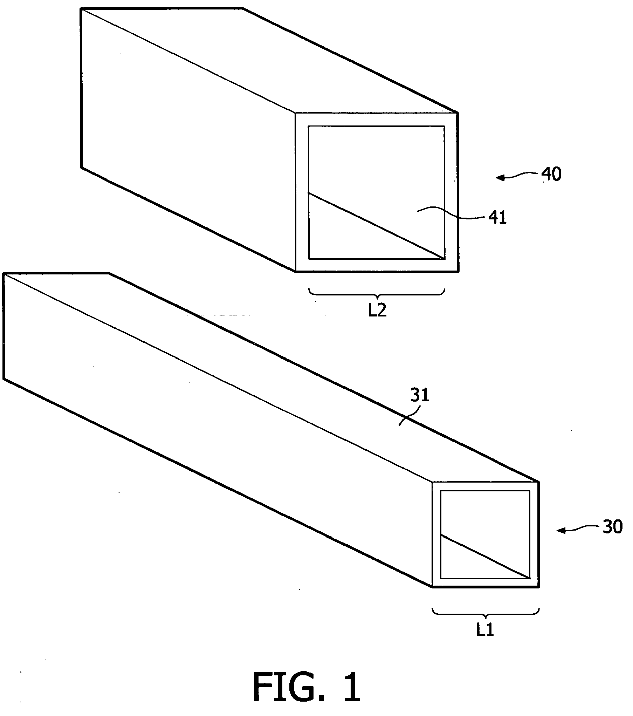

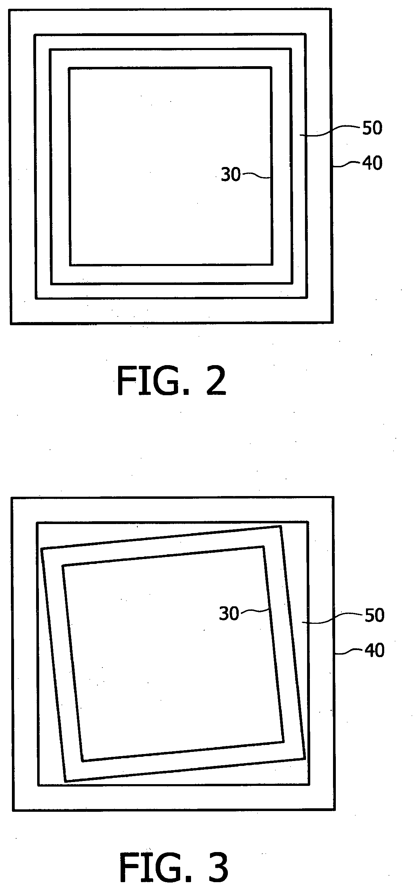

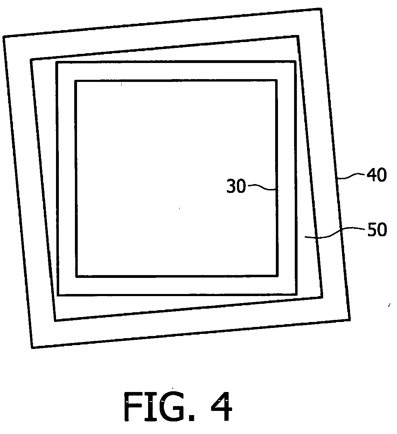

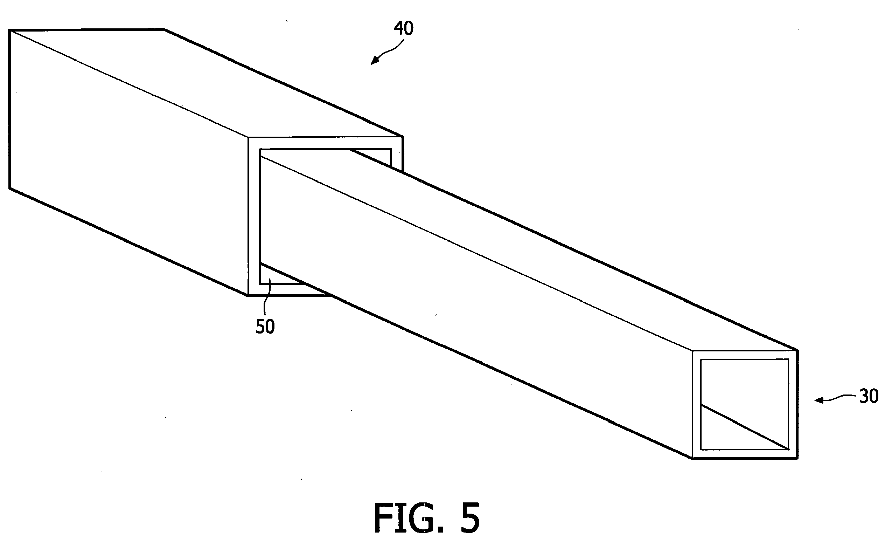

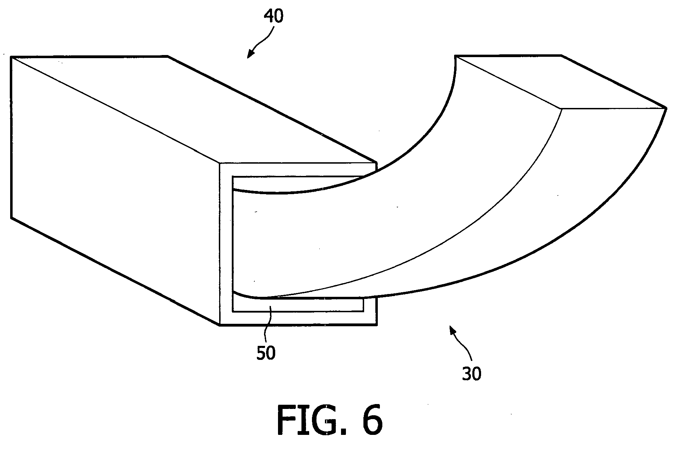

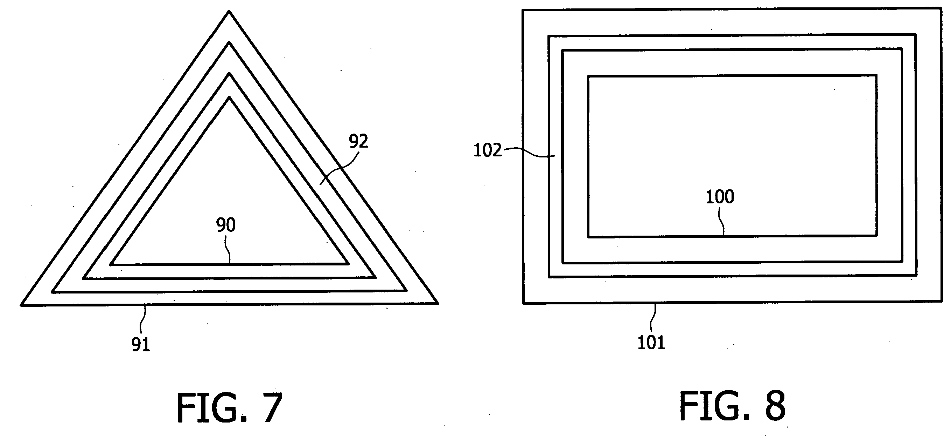

The present invention is premised on a nested pair of tubes having interlocking shapes to limit rotation of the tubes relative to a gap between the tubes. One benefit of this interlocking of the tubes is a fixed or consistent orientation of the inner tube relative to the outer tube as the inner tube is extended into or retracted from the outer tube. This benefit is particularly important in the context of the inner tube having a non-zero curvature (e.g., an arc).
For example, FIG. 1 illustrates an inner tube 30 and an outer tube 40 for purposes of demonstrating the premise of the present invention. Tubes 30 and 40 are configured and dimensioned to facilitate a nesting of inner tube 30 within outer tube 40 with a gap 50 between tubes 30 and 40 as shown in FIGS. 2-4. Gap 50 is required to facilitate a nesting of inner tube 30 within outer tube 40 with minimal friction. Tubes 30 and 40 have a square interlocking shape that limits rotation of tubes 30 and 40 relative to gap 50 as shown in FIGS. 2-4. More particularly, FIG. 2 illustrates a symmetrical nesting of inner tube 30 within outer tube 40, FIG. 3 illustrates a rotation of inner tube 30 within outer tube 40 that is limited by outer tube 40, and FIG. 4 illustrate a rotation of outer tube 40 about inner tube 30 that is limited by inner tube 30.
In practice, the gap between nested tubes will typically be small relative to the size of the tubes. However, tubes 30 and 40 are not drawn to scale for purposes of demonstrating the premise of the present invention. Nonetheless, FIGS. 2-4 exemplify a benefit of interlocking tubes 30 and 40 in achieving a consistent orientation of inner tube 30 relative to outer tube 40 as inner tube 30 is extended into or retracted from the outer tube 40. For example, FIG. 5 illustrates a consistent orientation of inner tube 30 relative to outer tube 40 with gap 50 therebetween in view of both tubes 30 and 40 having a zero curvature (i.e., straight) and FIG. 6 illustrates a consistent orientation of inner tube 30 relative to outer tube 40 with gap 50 therebetween in view of inner tube 30 having a non-zero curvature and outer tube 40 having a zero curvature.
In practice, a nested cannula set of the present invention employs two or more telescoping tubes with each tube having a pre-set interlocking shape and a pre-set curvature. For the outermost tube of the set, the pre-set interlocking shape is relevant for the inner surface of such tube. For the innermost tube of the set, the pre-set interlocking shape is relevant for the outer surface of such tube. For any intermediate tube of the set, the pre-set interlocking shape is relevant for both the external and outer surfaces of such tube.
Also in practice, the interlocking shape of each tube is any shape that interlocks an inner tube to an outer tube whenever the inner tube is nested within the outer tube whereby any individual rotation about the gap therebetween by the inner tube is limited by the outer tube and any individual rotation about the gap therebetween by the outer tube is limited by the inner tube. Such interlocking shapes for the tubes include, but are not limited to, a polygonal interlocking shape, a non-circular closed curve interlocking shape, a polygonal-closed curve interlocking shape, and a keyway interlocking shape. Yet another variety of interlocking shapes relies on non-scaled versions of a single shape, for example a rectangle or triangle interlocked within a hexagon.
For example, FIG. 7 illustrates a triangular interlocking shape of an inner tube 90 and an outer tube 91 with a gap 92 therebetween.
FIG. 8 illustrates a rectangular interlocking shape of an inner tube 100 and an outer tube 101 with a gap 102 therebetween.

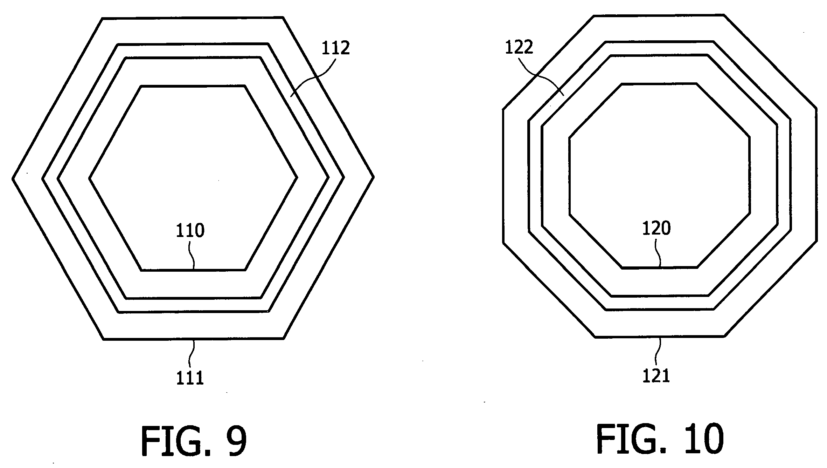

FIG. 9 illustrates a hexagonal interlocking shape of an inner tube 110 and an outer tube 111 with a gap 112 therebetween.

FIG. 10 illustrates an octagonal interlocking shape of an inner tube 120 and an outer tube 121 with a gap 122 therebetween.

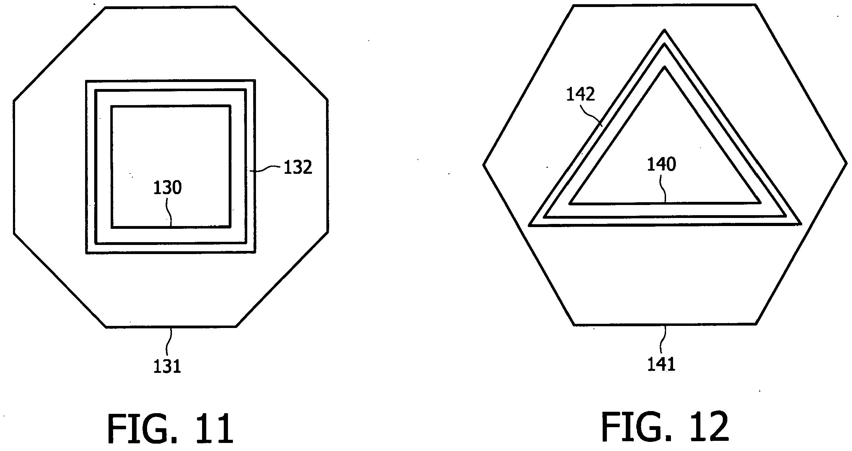

FIG. 11 illustrates an alternative square interlocking shape of an inner tube 130 and an outer tube with square inner shape and octagonal outer shape 131 with a gap 132 therebetween.

FIG. 12 illustrates an alternative triangular interlocking shape of an inner tube 140 and an outer tube with triangular inner shape and hexagonal outer shape 141 with a gap 142 therebetween.

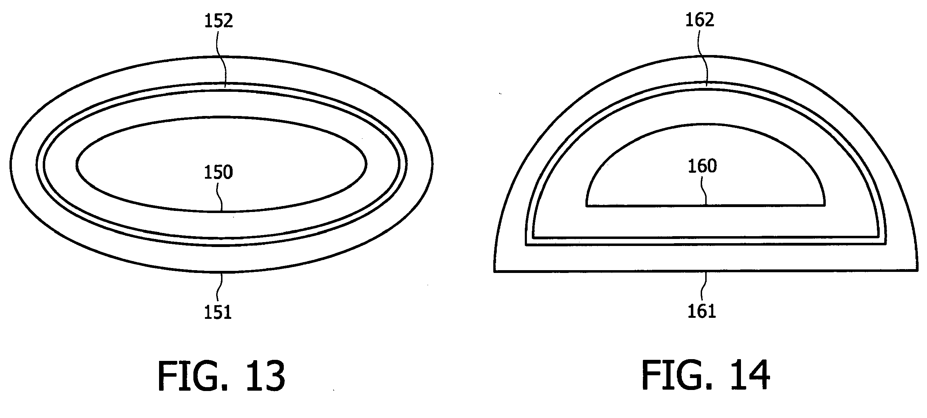

FIG. 13 illustrates an elliptical interlocking shape of an inner tube 150 and an outer tube 151 with a gap 152 therebetween.

FIG. 14 illustrates a semicircular interlocking shape of an inner tube 160 and an outer tube 161 with a gap 162 therebetween.

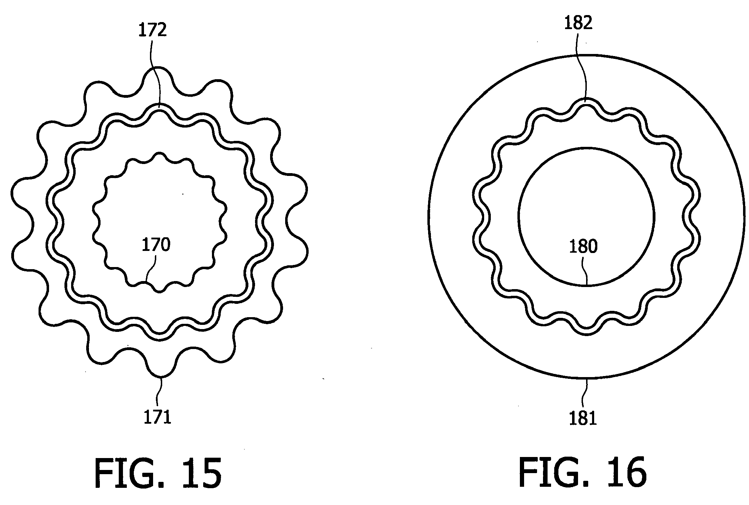

FIG. 15 illustrates a flute interlocking shape of a flute inner tube 170 and a flute outer tube 171 with a gap 172 therebetween.

FIG. 16 illustrates an alternative flute interlocking shape of an inner tube having a fluted outer shape and circular inner shape 180 and an outer tube having a fluted inner shape and circular outer shape 181 with a gap 182 therebetween.

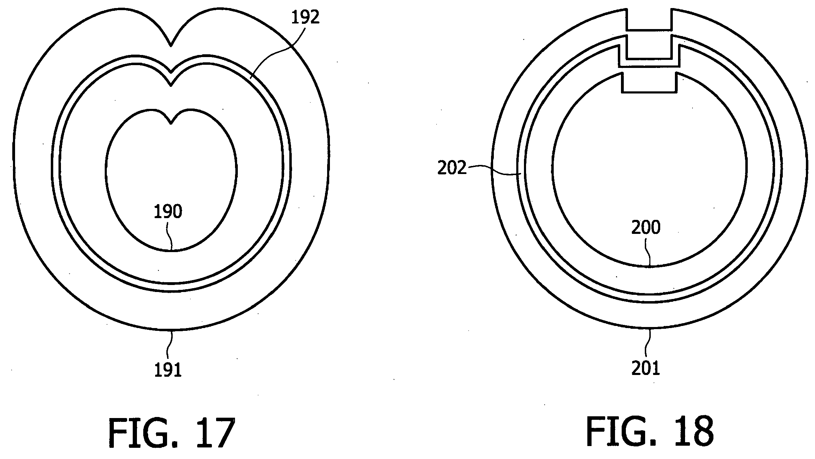

FIG. 17 illustrates a cardioid interlocking shape of an inner tube 190 and an outer tube 191 with a gap 192 therebetween.

FIG. 18 illustrates a keyway interlocking shape of an inner tube 200 and an outer tube 201 with a gap 202 therebetween.

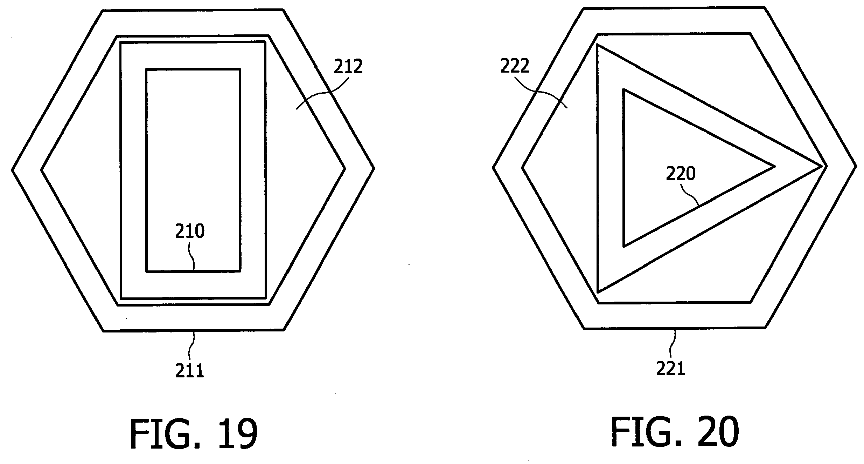

FIG 19 illustrates a rectangular interlocking shape of an inner tube 210 and an outer hexagonal tube 211 with a gap 212 therebetween.

FIG 20 illustrates a triangular interlocking shape of an inner tube 220 and an outer hexagonal tube 221 with a gap 222 therebetween.

Referring to FIGS. 5, 7, 9-12 and 20, each of the illustrated polygon interlocking shapes have an N number of equal sides of the larger locking polygon, wherein N > 2. In practice, compliance with the following equations [1] and [2] as associated with corresponding sides of such tubes facilitates an interlocking of the tubes in accordance with the present invention:

$$OS_{IT}/IS_{OT} > K \qquad\qquad [1]$$

$$K = \cos(\pi/N) \qquad\qquad [2]$$

where $OS_{IT}$ is the length of each outer side of the inner tube, $IS_{OT}$ is the length of each inner side of outer tube, and N is the number of sides of the inside of the larger polygonal tube. For example, referring to FIG. 1, a ratio of a length L1 of each outer side 31 of inner tube 30 to a length L2 of each inner side 41 of outer tube 40 must be equal to or great than factor K based on N = 4. In FIG. 9 for example, N=6, therefore K= cos($\pi$ /6) = sqrt(3) / 2 or about 86.6%. This means that the outer side of the inner tube must be at least 86.6% of the length of the inner side of the outer tube in order to interlock. Clearly, as the number approaches 100%, there is a smaller gap, and lower error in possible rotation.

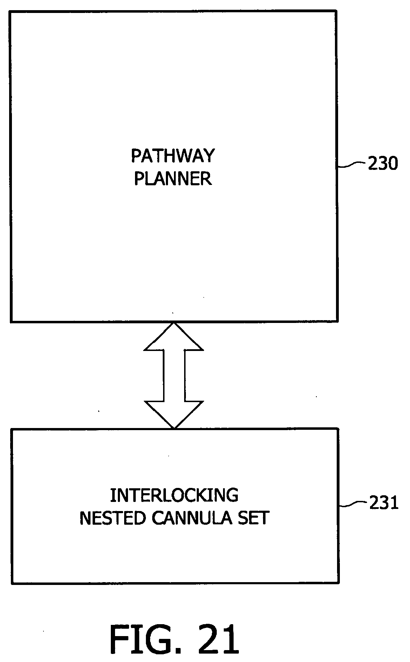

FIG. 21 illustrates a pathway planner 230 as known in the art for designing a plurality of telescoping tubes with configured and dimensioned with pre-set shapes and curvatures. Pathway lanner 230 specifies the specific lengths that the tubes are extended to reach a target location relative to an anatomical region. Specifically, pathway planner 230 uses a neighborhood of arc and straight threads to encapsulate a set of fundamental motions of a nested set of interlocking tubes 231 of the present invention that can be performed in free space based on available controls and mechanical properties of the tubes 231, and more particularly, based on the available fixed orientations between nested tubes 231. Based on the neighborhood, pathway planner 230 defines the extension of each tube to achieve a specific length, and the orientation of each tube relative to the previous tube.

An example set of tubes might be specified as follows, wherein the term thread is used to describe the selected arc having a specific tube orientation relative to the prior tube, and the length is the extension of the current tube relative to the prior tube:

Number of tubes needed for this path is: 8
Tube number 1 : length= 17.4994mm, thread = 6
Tube number 2 : length= 63mm, thread = 0
Tube number 3 : length= 7.49973mm, thread = 1
Tube number 4 : length= 28.5mm, thread = 0
Tube number 5 : length= 7.99971 mm, thread = 5

Tube number 6 : length= 7.5mm, thread = 0
Tube number 7 : length= 1.99993mm, thread = 4
Tube number 8 : length= 3.5mm, thread = 0

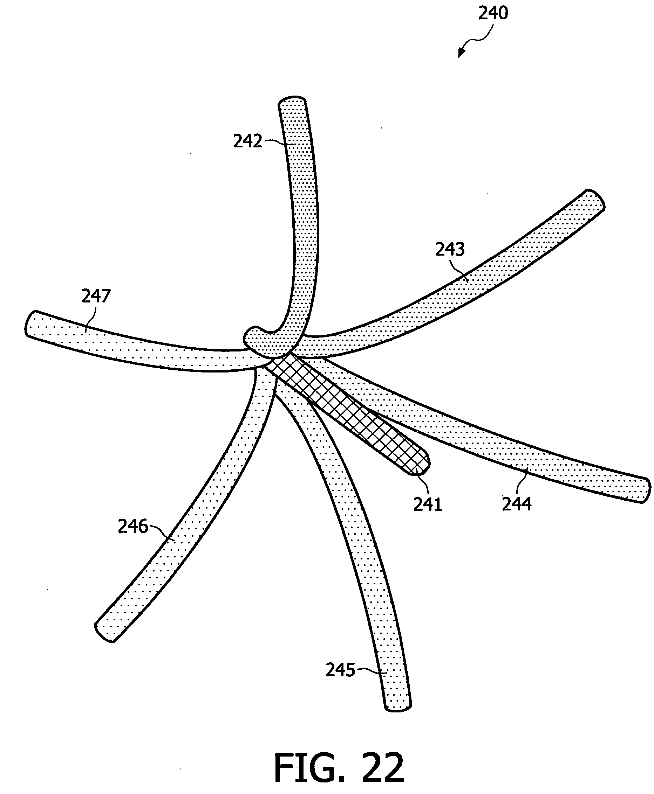

Generally, a neighborhood may have discrete rotational arcs in view of the fact that discrete rotational symmetries minimize the number of pre-manufactured tubes by providing multiple ways to use each tube. For example, FIG. 22 illustrates an exemplary neighborhood 240 having a straight thread 241 and six (6) 14mm turning radius arcs 242-247. Each of the arcs 242-247 can be extended to any length, following the same curvature. Each arc is preferably short enough so that the arc does not return to the same point (position and orientation). The optimal interlocking shape for the tubes 231 (FIG. 21) resulting from this neighborhood 240 is a hexagonal interlocking shape corresponding to the discrete rotational symmetry of arcs 242-247, which would yield six (6) settable angles for each nested tube 231.

Hexagonal tubing can be formed by extrusion, casting, creasing, drawing, forming and shrinking. The extrusion process is accomplished by pushing molten material through a die with the desired tubes shape. Casting is accomplished by cooling molten material held within a mold. Creasing is accomplished by pressing deformable tube to create the desired corners; a roughly hexagonal shape can thus be created by pressing the originally circular tube flat three times (each time the tube is by rotated sixty degrees). Another form of manufacturing hexagonal tubes using creasing is to introduce five 120 degree creases in a sheet of material and to weld the two ends of the sheet together. Forming is accomplished by heating a deformable material and constraining it to take the desired hexagonal shape. Shrinking is accomplished by heating heat shrink tubing around a hexagonal form. Though extrusion followed by drawing is an exemplary process for large-scale production, prototypes can be made by using the shrinking method.

Often it is desirable to curve each of the tubes. This is performed by shaping the die to create curved tubing by: generating a curved mold, or creasing an already curved circular tube, or forming onto or with a curved mold, or shrinking onto a curved form. Curving the tube can also be done after the hexagonal shape has been made by heating the material and constraining its path to the desired curve. An exemplary method for curving drawn tubes is to deform the tubes at ambient temperature. An exemplary method of curving shrink tubes is to create the tubes around an already curved mandrel.

The cannula may consist of any single material, or of a composite of multiple materials. The desired materials will depend on the application and the manufacturing processes that are available. Often flexible materials that can support their own weight and the weight of the payload without considerable deflection under the gravitational force are desired. If the cannula must apply forces at its tip or along its surface, the cannula constructed should be rigid enough to apply these forces without considerable deflection. It is also desirable for the tube to be firm enough to hold its shape; if the tube deforms too readily the cannulas may not hold their angles. When the tubes are to be translated with respect to one another it is desirable to select tube materials that minimize friction along the interface. Some materials and applications may require an intercannular lubricant to reduce the frictional resistance. For surgical application is also important that the material be fit for internal human contact. Additionally, some surgical applications require a non-ferromagnetic material to allow MRI imaging during the procedure. For flexible surgical applications that also require very small cannula diameters, or when significant forces are present, autoclavable superelastic nickel titanium alloys may be used. For other applications a wide variety of polymers may be used. These include, but are not limited to Polycarbonate, Nylon, Polypropylene, Polyolefins, and Teflon PTFE.

While various embodiments of the present invention have been illustrated and described, it will be understood by those skilled in the art that the methods and the system as described herein are illustrative, and various changes and modifications may be made and equivalents may be substituted for elements thereof without departing from the true scope of the present invention. In addition, many modifications may be made to adapt the teachings of the present invention to entity path planning without departing from its central scope. Therefore, it is intended that the present invention not be limited to the particular embodiments disclosed as the best mode contemplated for carrying out the present invention, but that the present invention include all embodiments falling within the scope of the appended claims.

**Claims**

1. An interlocking nested cannula set (231), comprising:

   a plurality of telescoping tubes cooperatively configured and dimensioned to reach a target location relative to an anatomical region,

   wherein each tube has an interlocking shape, and

   wherein a nesting of an inner tube (30) within an outer tube (40) includes

a gap (50) between the inner tube (30) and the outer tube (40) for allowing a rotation of the inner tube (30) within the outer tube (40) that is limited by the outer tube (40) and a rotation of the outer tube (40) about the inner tube (30) that is limited by the inner tube (30), and
the inner tube (30) and the outer tube (40) interlocking within the gap (50) to limit the rotation of the inner tube (30) and the outer tube (40) relative to the gap (50).

2. The interlocking nested cannula set (231) of claim 1, wherein at least one of the inner tube (30) and the outer tube (40) has a polygonal interlocking shape.

3. The interlocking nested cannula set (231) of claim 1, wherein at least one of the inner tube (30) and the outer tube (40) has a non-circular closed curve interlocking shape.

4. The interlocking nested cannula set (231) of claim 1, wherein at least one of the inner tube (30) and the outer tube (40) has a polygonal-closed curve hybrid interlocking shape.

5. The interlocking nested cannula set (231) of claim 1, wherein at least one of the inner tube (30) and the outer tube (40) has a keyway interlocking shape.

6. The interlocking nested cannula set (231) of claim 5, wherein an interlocking shape of the inner tube (30) and the interlocking shape of the outer tube (40) are identical.

7. The interlocking nested cannula set (231) of claim 5, wherein the interlocking shape of the inner tube (30) and the interlocking shape of the outer tube (40) are different.

8. An interlocking nested cannula system, comprising:

   a pathway planner (230) for designing an interlocking nested cannula set (231) of telescoping tubes cooperatively configured and dimensioned to reach a target location relative to an anatomical region,

   wherein each tube has an interlocking shape, and

   wherein a nesting of an inner tube (30) within an outer tube (40) includes

   a gap (50) between the inner tube (30) and the outer tube (40) for allowing a rotation of the inner tube (30) within the outer tube (40) that is limited by the outer tube (40) and a rotation of the outer tube (40) about the inner tube (30) that is limited by the inner tube (30), and
   an outer surface (31) of the inner tube (30) and an inner surface (42) of the outer tube (40) interlocking within the gap (50) to limit the rotation of the inner tube (30) and the outer tube (40) relative to the gap (50).

9. The interlocking nested cannula system of claim 8, wherein at least one of the inner tube (30) and the outer tube (40) has a polygonal interlocking shape.

10. The interlocking nested cannula system of claim 8, wherein at least one of the inner tube (30) and the outer tube (40) has a non-circular closed curve interlocking shape.

11. The interlocking nested cannula system of claim 8, wherein at least one of the inner tube (30) and the outer tube (40) has a polygonal-closed curve hybrid interlocking shape.

12. The interlocking nested cannula system of claim 8, wherein at least one of the inner tube (30) and the outer tube (40) has a keyway interlocking shape.

13. The interlocking nested cannula system of claim 8, wherein an interlocking shape of the inner tube (30) and the interlocking shape of the outer tube (40) are identical.

14. The interlocking nested cannula system of claim 8, wherein the interlocking shape of the inner tube (30) and the interlocking shape of the outer tube (40) are different.

15. The interlocking nested cannula system of claim 8,

wherein the pathway planner (230) is operable to use a neighborhood (240) having a discrete rotational set of arcs (242-247) to encapsulate a set of motions of the tubes relative to a set location; and

wherein the pathway planner (230) is further operable to define the relative orientation for assembling the tubes based on the selected arcs of the neighborhood (240), and the extension required for each tube.

**Patentansprüche**

1. Verschachtelter ineinandergreifender Kanülensatz (231), der Folgendes umfasst:

    eine Vielzahl von ineinanderschiebbaren Röhren, die gemeinsam konfiguriert und bemessen sind, um einen Zielort in Bezug auf eine anatomische Region zu erreichen,

    wobei jede Röhre eine ineinandergreifende Form hat, und

    wobei eine Verschachtelung einer inneren Röhre (30) mit einer äußeren Röhre (40) einen Spalt (50) zwischen der inneren Röhre (30) und der äußeren Röhre (40) umfasst, um eine Drehung der inneren Röhre (30) in der äußeren Röhre (40), die durch die äußere Röhre (40) begrenzt wird, und eine Drehung der äußeren Röhre (40) um die innere Röhre (30), die durch die innere Röhre (30) begrenzt wird, zu ermöglichen, und

    die innere Röhre (30) und die äußere Röhre (40) innerhalb des Spalts (50) ineinandergreifen, um die Drehung der inneren Röhre (30) und der äußeren Röhre (40) in Bezug auf den Spalt (50) zu begrenzen.

2. Verschachtelter ineinandergreifender Kanülensatz (231) nach Anspruch 1, wobei mindestens entweder die innere Röhre (30) oder die äußere Röhre (40) eine polygonale ineinandergreifende Form hat.

3. Verschachtelter ineinandergreifender Kanülensatz (231) nach Anspruch 1, wobei mindestens entweder die innere Röhre (30) oder die äußere Röhre (40) eine ineinandergreifende Form einer nicht-kreisförmigen, geschlossenen Kurve hat.

4. Verschachtelter ineinandergreifender Kanülensatz (231) nach Anspruch 1, wobei mindestens entweder die innere Röhre (30) oder die äußere Röhre (40) eine ineinandergreifende Form eines Hybrids aus Polygon und geschlossener Kurve hat.

5. Verschachtelter ineinandergreifender Kanülensatz (231) nach Anspruch 1, wobei mindestens entweder die innere Röhre (30) oder die äußere Röhre (40) eine ineinandergreifende Form einer Keilnut hat.

6. Verschachtelter ineinandergreifender Kanülensatz (231) nach Anspruch 5, wobei eine ineinandergreifende Form der inneren Röhre (30) und die ineinandergreifende Form der äußeren Röhre (40) identisch sind.

7. Verschachtelter ineinandergreifender Kanülensatz (231) nach Anspruch 5, wobei die ineinandergreifende Form der inneren Röhre (30) und die ineinandergreifende Form der äußeren Röhre (40) unterschiedlich sind.

8. Verschachteltes ineinandergreifendes Kanülensystem, das Folgendes umfasst:

    einen Pfadplaner (230) zum Gestalten eines ineinandergreifenden verschachtelten Kanülensatzes (231) von ineinanderschiebbaren Röhren, die gemeinsam konfiguriert und bemessen sind, um einen Zielort in Bezug auf eine anatomische Region zu erreichen,

    wobei jede Röhre eine ineinandergreifende Form hat, und

    wobei eine Verschachtelung einer inneren Röhre (30) mit einer äußeren Röhre (40) einen Spalt (50) zwischen der inneren Röhre (30) und der äußeren Röhre (40) umfasst, um eine Drehung der inneren Röhre (30) in der äußeren Röhre (40), die durch die äußere Röhre (40) begrenzt wird, und eine Drehung der äußeren Röhre (40) um die innere Röhre (30), die durch die innere Röhre (30) begrenzt wird, zu ermöglichen, und

    eine Außenfläche (31) der inneren Röhre (30) und eine Innenfläche (42) der äußeren Röhre (40) innerhalb des Spalts (50) ineinandergreifen, um die Drehung der inneren Röhre (30) und der äußeren Röhre (40) in Bezug auf den Spalt (50) zu begrenzen.

9. Verschachteltes ineinandergreifendes Kanülensystem nach Anspruch 8, wobei mindestens entweder die innere Röhre (30) oder die äußere Röhre (40) eine polygonale ineinandergreifende Form hat.

10. Verschachteltes ineinandergreifendes Kanülensystem nach Anspruch 8, wobei mindestens entweder die innere Röhre (30) oder die äußere Röhre (40) eine ineinandergreifende Form einer nicht-kreisförmigen, geschlossenen Kurve hat.

11. Verschachteltes ineinandergreifendes Kanülensystem nach Anspruch 8, wobei mindestens entweder die innere Röhre (30) oder die äußere Röhre (40) eine ineinandergreifende Form eines Hybrids aus Polygon und geschlossener Kurve hat.

12. Verschachteltes ineinandergreifendes Kanülensystem nach Anspruch 8, wobei mindestens entweder die innere Röhre (30) oder die äußere Röhre (40) eine ineinandergreifende Form einer Keilnut hat.

13. Verschachteltes ineinandergreifendes Kanülensystem nach Anspruch 8, wobei eine ineinandergreifende Form der inneren Röhre (30) und die ineinandergreifende Form der äußeren Röhre (40) identisch sind.

14. Verschachteltes ineinandergreifendes Kanülensystem nach Anspruch 8, wobei die ineinandergreifende Form der inneren Röhre (30) und die ineinandergreifende Form der äußeren Röhre (40) unterschiedlich sind.

15. Verschachteltes ineinandergreifendes Kanülensystem nach Anspruch 8,
wobei der Pfadplaner (230) betriebsfähig ist, um eine Nachbarschaft (240) mit einem diskreten Rotationsbogensatz (242-247) zu verwenden, um einen Satz von Bewegungen der Röhren in Bezug auf einen vorgegebenen Ort einzukapseln; und
wobei der Pfadplaner (230) weiterhin betriebsfähig ist, um die relative Ausrichtung zum Zusammenbauen der Röhren basierend auf den ausgewählten Bögen der Nachbarschaft (240) und die für jede Röhre benötigte Verlängerung zu definieren.


**Revendications**

1. Jeu de canules emboîtées d'interverrouillage (231), comprenant :

   une pluralité de tubes télescopiques configurés de manière coopérative et dimensionnés pour atteindre un emplacement cible par rapport à une région anatomique,

   dans lequel chaque tube présente une forme d'interverrouillage, et

   dans lequel un emboîtement d'un tube intérieur (30) dans un tube extérieur (40) comprend

   un espace (50) entre le tube intérieur (30) et le tube extérieur (40) pour permettre une rotation du tube intérieur (30) dans le tube extérieur (40) qui est limitée par le tube extérieur (40) et une rotation du tube extérieur (40) autour du tube intérieur (30) qui est limitée par le tube intérieur (30), et
   le tube intérieur (30) et le tube extérieur (40) s'interverrouillant dans l'espace (50) afin de limiter la rotation du tube intérieur (30) et du tube extérieur (40) par rapport à l'espace (50).

2. Jeu de canules emboîtées d'interverrouillage (231) selon la revendication 1, dans lequel au moins un tube parmi le tube intérieur (30) et le tube extérieur (40) présente une forme d'interverrouillage polygonale.

3. Jeu de canules emboîtées d'interverrouillage (231) selon la revendication 1, dans lequel au moins un tube parmi le tube intérieur (30) et le tube extérieur (40) présente une forme d'interverrouillage à courbe fermée non circulaire.

4. Jeu de canules emboîtées d'interverrouillage (231) selon la revendication 1, dans lequel au moins un tube parmi le tube intérieur (30) et le tube extérieur (40) présente une forme d'interverrouillage hybride à courbe fermée polygonale.

**5.** Jeu de canules emboîtées d'interverrouillage (231) selon la revendication 1, dans lequel au moins un tube parmi le tube intérieur (30) et le tube extérieur (40) présente une forme d'interverrouillage à rainure de clavette.

**6.** Jeu de canules emboîtées d'interverrouillage (231) selon la revendication 5, dans lequel une forme d'interverrouillage du tube intérieur (30) et la forme d'interverrouillage du tube extérieur (40) sont identiques.

**7.** Jeu de canules emboîtées d'interverrouillage (231) selon la revendication 5, dans lequel la forme d'interverrouillage du tube intérieur (30) et la forme d'interverrouillage du tube extérieur (40) sont différentes.

**8.** Système de canules emboîtées d'interverrouillage, comprenant :

un planificateur (230) de voies pour concevoir un jeu de canules emboîtées d'interverrouillage (231) de tubes télescopiques configurés de manière coopérative et dimensionnés pour atteindre un emplacement cible par rapport à une région anatomique,

dans lequel chaque tube présente une forme d'interverrouillage, et

dans lequel un emboîtement d'un tube intérieur (30) dans un tube extérieur (40) comprend

un espace (50) entre le tube intérieur (30) et le tube extérieur (40) pour permettre une rotation du tube intérieur (30) dans le tube extérieur (40) qui est limitée par le tube extérieur (40) et une rotation du tube extérieur (40) autour du tube intérieur (30) qui est limitée par le tube intérieur (30), et
une surface extérieure (31) du tube intérieur (30) et une surface intérieure (42) du tube extérieur (40) s'interverrouillant dans l'espace (50) afin de limiter la rotation du tube intérieur (30) et du tube extérieur (40) par rapport à l'espace (50).

**9.** Système de canules emboîtées d'interverrouillage selon la revendication 8, dans lequel au moins un tube parmi le tube intérieur (30) et le tube extérieur (40) présente une forme d'interverrouillage polygonale.

**10.** Système de canules emboîtées d'interverrouillage selon la revendication 8, dans lequel au moins un tube parmi le tube intérieur (30) et le tube extérieur (40) présente une forme d'interverrouillage à courbe fermée non circulaire.

**11.** Système de canules emboîtées d'interverrouillage selon la revendication 8, dans lequel au moins un tube parmi le tube intérieur (30) et le tube extérieur (40) présente une forme d'interverrouillage hybride à courbe fermée polygonale.

**12.** Système de canules emboîtées d'interverrouillage selon la revendication 8, dans lequel au moins un tube parmi le tube intérieur (30) et le tube extérieur (40) présente une forme d'interverrouillage à rainure de clavette.

**13.** Système de canules emboîtées d'interverrouillage selon la revendication 8, dans lequel une forme d'interverrouillage du tube intérieur (30) et la forme d'interverrouillage du tube extérieur (40) sont identiques.

**14.** Système de canules emboîtées d'interverrouillage selon la revendication 8, dans lequel la forme d'interverrouillage du tube intérieur (30) et la forme d'interverrouillage du tube extérieur (40) sont différentes.

**15.** Système de canules emboîtées d'interverrouillage selon la revendication 8,
dans lequel le planificateur (230) de voies sert à utiliser un quartier (240) ayant un ensemble rotatif distinct d'arcs (242-247) pour encapsuler un jeu de mouvements des tubes par rapport à un emplacement déterminé ; et
dans lequel le planificateur (230) de voies sert en outre à définir l'orientation relative pour assembler les tubes sur la base des arcs sélectionnés du quartier (240), et l'extension nécessitée pour chaque tube.

FIG. 1

FIG. 2

FIG. 3

FIG. 4

FIG. 5

FIG. 6

FIG. 7

FIG. 8

EP 2 346 420 B1

FIG. 9

FIG. 10

**FIG. 11**

**FIG. 12**

152

150

151

**FIG. 13**

162

160

161

**FIG. 14**

EP 2 346 420 B1

FIG. 16

FIG. 15

EP 2 346 420 B1

FIG. 17

FIG. 18

FIG. 19

FIG. 20

PATHWAY
PLANNER

~230

INTERLOCKING
NESTED CANNULA SET

~231

FIG. 21

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007008332 A2
- WO 2007059233 A2
- US 20030199916 A1
- WO 2008032230 A